# EUROPEAN PATENT APPLICATION

(11) **EP 4 528 749 A1**
(43) Date of publication of application: **26.03.2025**
(21) Application number: 23199273.6
(22) Date of filing: 25.09.2023
(51) Int. Cl.: G16H 40/63, G16H 50/20

(54) **PHYSIOLOGICAL PARAMETER REPRESENTATION**

(71) Applicant: Koninklijke Philips N.V., 5656 AG Eindhoven (NL)
(72) Inventor: VAN LIESHOUT, Ron Martinus Laurentius, Eindhoven (NL); HUIJBREGTS, Laurentia Johanna, Eindhoven (NL); BOUTS, Mark Jacobus Rosalie Joseph, 5656AG Eindhoven (NL); BRANS, Harold Johannes Antonius, Eindhoven (NL); BUIZZA, Roberto, Eindhoven (NL)
(74) Representative: Philips Intellectual Property & Standards

(57) **Abstract**

Disclosed concepts aim to provide schemes, solutions, concepts, designs, methods, and systems pertaining to displaying a graphical representation of values of a physiological parameter. Specifically, a consecutive set of values is identified, having values that differ/deviate from values immediately preceding and/or following the consecutive set. In other words, short term deviations of the value of the physiological parameter are identified, which may contribute to a cluttered and confusing display of the physiological parameter. Therefore, the identified consecutive set of values are analyzed with respect to a ruleset to determine a clinical relevance of the values, and the graphical representation is modified accordingly. For example, if the consecutive set of values is of low clinical relevance, then graphical parameters of the representation of the set of values may be modified to make the representation less visible to a viewer.

## Description

### FIELD OF THE INVENTION

The present invention relates to the field of subject monitoring, and more particularly to graphical representation of subject physiological parameters.

### BACKGROUND OF THE INVENTION

A view of a data stream of subject physiological parameters are often provided to caregivers to understand the condition of the subject, and how their condition has changed over time. Physiological parameters include vital signs (e.g., heart rate, respiration rate, and blood pressure), advanced hemodynamic parameters (e.g., cardiac output, stroke volume, and systemic vascular resistance), haematological parameters (e.g., red and white blood cell counts and haemoglobin concentration), and analyte concentrations (e.g., glucose or lactate concentrations in whole blood, plasma, or serum).

With so many potentially relevant physiological parameters to present to caregivers, screens displaying such parameters can often look cluttered. This problem is compounded when there are short-term changes in parameters relative to the timeline being displayed. As a result, interpretation of the screen can be complex, resulting in high cognitive load for caregivers. In a worst case scenario, relevant changes in physiological parameters may be missed, resulting in poor outcomes for subjects.

Some methods are employed to reduce this cognitive load, such as only providing visualizations of physiological parameters relating to a condition of the subject. For example, parameters related to the haemodynamic status of the subject may be prioritized for subjects suffering from haemodynamic instability. Moreover, different colors may be used to represent the range of the physiological parameter, or an indication of the recent trend of the physiological parameter may be provided on the display.

Nevertheless, there exists a need for methods of visualizing all important physiological parameters for easy comparison that do not require undue cognitive burden for a caregiver to interpret.

### SUMMARY OF THE INVENTION

The invention is defined by the claims.

According to examples in accordance with an aspect of the invention, there is provided a method for displaying a graphical representation of values of a physiological parameter of a subject as a function of time.

The method comprises receiving a data series of values of the physiological parameter;
identifying a consecutive set of the values of the physiological parameter in a value range differing from a value range of the values of the physiological parameter immediately preceding and following the consecutive set of values, wherein the identified consecutive set of values span a length of time below a predetermined minimum length of time;
analyzing the consecutive set of values based on a ruleset to determine a clinical relevance of the consecutive set of values; and
modifying the graphical representation of the consecutive set of values of the physiological parameter based on the determined clinical relevance.

Proposed concepts pertain to displaying a graphical representation of values of a physiological parameter. Specifically, a consecutive set of values are identified, having values that differ/deviate from values immediately preceding and/or following the consecutive set. In other words, short term deviations of the value of the physiological parameter are identified, which may contribute to a cluttered and confusing display of the physiological parameter. Therefore, the identified consecutive set of values are analyzed with respect to a ruleset to determine a clinical relevance of the values, and the graphical representation is modified accordingly. For example, if the consecutive set of values are of low clinical relevance, then graphical parameters of the representation of the set of values may be modified to make the representation less visible to a viewer.

In other words, disclosed concepts attempt to improve an ease by which an observer may interpret a graphical representation of a data series of values of a physiological parameter. This is achieved by first identifying (at least one) consecutive set of values within the data series belonging to a data range different from the data range of neighboring values. This may initially appear as a brief spike or trough in the graphical representation of the physiological parameter, standing out from the neighboring values for a brief period (i.e. less than a predetermined minimum length of time).

Then, a clinical relevance/significance of the identified consecutive set of values is assessed, which may be based on circumstances of the subject, a context of the acquisition of the values, or a reaction/action of caregiver(s) to the set of values, for example. If of high clinical relevance, then the graphical representation of the set of values may be adapted to be more easily visible, thereby ensuring observers attention is drawn to this relevant variation in the physiological parameter. If of low clinical relevance, then the graphical representation of the set of values may be adapted to be less noticeable (or entirely invisible) to the observer, therefore ensuring that the observer can focus on more relevant aspects of the visual representation of the data series.

The data series of values of the physiological parameter may be acquired directly from sensors on the subject, or obtained indirectly from EMR or from a processor configured to process signals from sensors to output a physiological parameter value. That is, the data series of values may be measured values, or calculated values.

The predetermined minimum length of time may not be fixed. The predetermined length of time may be set by a user, or may be determined based on the physiological parameter and rules set by the user. Further, the predetermined minimum length of time may be dependent on a zoom factor on the timescale (i.e., the length of time spanned by the displayed physiological parameter). The predetermined minimum length of time may be relative to the length of time being spanned by the displayed physiological parameter values (e.g., 30 seconds when 30 minutes of the physiological parameter is displayed, or 5 minutes when 8 hours of the physiological parameter is displayed).

In other words, some embodiments provide that the predetermined minimum length of time may be based on the length of time spanned by the displayed values (i.e. a zoom factor or time scale) of the physiological parameter.

In essence, implementations of the invention overcome problems associated with simply outputting a series of measured or generated values of a physiological parameter to a screen. Often, observers need to commit high levels of cognitive effort to identify parts of the data series that are of interest to them, being distracted by variations in the physiological parameter that may be of very low clinical significance (e.g., a brief spike in heart rate due to brief subject movement, or expected changes in glucose concentration after an intervention). Alternatively, clinically relevant changes in a physiological parameter may be missed as they may only last very briefly in comparison to the timescale of the data series on display. This problem is compounded when many different physiological parameters are provided on one display. Implementations of this invention may overcome these issues by identifying and classifying short-term variations, and modifying the graphical representation accordingly.

In some embodiments, identifying the consecutive set of values of the physiological parameter may comprise obtaining a set of value ranges, each describing a discrete numerical range of values for the physiological parameter; dividing the values of the physiological parameter into a plurality of data segments, each data segment comprising a consecutive set of values falling within one of the set of value ranges; and, for each data segment, determining whether the data segment spans a length of time below the predetermined minimum length of time.

This discretization of the data series of values of the physiological parameter provides a straightforward way in which to identify set(s) of consecutive values that span a length of time below the predetermined minimum length of time. Indeed, some existing means for displaying physiological parameters already complete this step, meaning that fewer modifications will need to be made to implement the invention.

Each of the set of value ranges may correspond to a different alarm setting related to the physiological parameter.

Accordingly, some embodiments are concerned with identifying when the values of the physiological parameter briefly correspond to an alarm setting, and determining the clinical significance of this brief change in alarm setting so as to appropriately display the graphical representation of the values.

An alarm setting may be defined as a (contextual) status of the physiological parameter (e.g., very low, low, normal, high and very high). If the physiological parameter is in a certain alarm setting, varying outputs may be provided to inform concerned parties (e.g., a sound provided to a caregiver).

More particularly, the set of the value ranges may comprise at least one value range corresponding to each of a normal range of the physiological parameter, an elevated range of the physiological parameter, a depressed range of the physiological parameter, a dangerously high range of the physiological parameter, and a dangerously low range of the physiological parameter.

The normal, elevated, depressed, dangerously high and dangerously low ranges of the physiological parameters may be defined by protocol, a user/clinician, from literature and/or historical population data.

In some embodiments, analyzing the consecutive set of values to determine the clinical relevance may comprise at least one of
comparing the consecutive set of values to an alarm condition describing values indicative of a potential health risk for the subject;
comparing the consecutive set of values to a goal condition describing target values of the physiological parameter of the subject;
analyzing a surrounding environment of the subject corresponding to the acquisition of the consecutive set of values;
analyzing a user action responsive to an alarm associated with the consecutive set of values;
analyzing a subject action performed by the subject or by a user during acquisition of the consecutive set of values; and
analyzing a length of time spanned by the displayed values of the physiological parameter.

Each of the above factors may impact whether the consecutive set of values is of clinical relevance and/or the extent of the clinical relevance.

For example, if during acquisition of the consecutive set of values the subject was moving, and this caused an alarm which was silenced by a clinician, then the consecutive set of values may be considered of low clinical significance. Or, if the consecutive set of values were associated with an intervention performed on them (e.g., they took medication), then the clinical relevance may be considered low in some circumstances.

The graphical representation of each of the values may be defined by a configurable set of graphical parameters. In this case, responsive to determining that the consecutive set of values are of low clinical relevance, modifying the graphical representation of the consecutive set of values may comprise modifying at least one of the graphical parameters of the consecutive set of values to be substantially equal to the graphical parameters of at least one of the values immediately preceding or following the consecutive set of values.

In other words, the graphical representation of each of the values in the data series corresponds to various settings dictating the visual characteristics of the graphical representation. Such graphical parameters determine the appearance of each value, and if set equal will look more similar/will be harder to differentiate. Therefore, if the consecutive set of values are of low clinical relevance, the visual representation of the values can be altered to match at least one of the neighboring values visual representations to thus be less noticeable.

In some case, the method may further comprise determining whether the values immediately preceding and following the consecutive set of values differ by less than a threshold amount. Then, responsive to determining that the values immediately preceding and following differ by less than the threshold amount, modifying the at least one of the graphical parameters may comprise modifying at least one of the graphical parameters of the consecutive set of values to be substantially equal to the graphical parameters of both of the values immediately preceding and following the consecutive set of values.

In essence, this is a check that the preceding and following values are similar enough that the graphical representation of the consecutive set of values therebetween can be set equal to both to thereby not stand out to an observer.

The threshold amount may be a system default, or may be a pre-defined configuration, or may be determined from protocol, set by a user/clinician, may be determined from literature and/or historical population data.

The configurable set of graphical parameters may comprise at least one of a color parameter, pattern parameter, elevation parameter, brightness parameter, and/or size parameter.

In some embodiments, responsive to determining that the consecutive set of values of the physiological parameter are of high clinical relevance, modifying the graphical representation of the consecutive set of values may comprise increasing a size parameter defining a size of the graphical representation of the consecutive set of values.

In other words, the graphical parameters of the consecutive set of values corresponding to size of the graphical representation of the consecutive set of values is modified so as to make the graphical representation larger. In this way, the consecutive set of values that may be relatively difficult for an observer to identify (due to spanning a short length of time) will be made to stand out more, drawing the attention of an observer to a highly clinically relevant event.

Specifically, increasing the size parameter of the consecutive set of values may comprise enlarging a length of the time axis of the graphical representation corresponding to the consecutive set of values.

In effect, this means that the consecutive set of values are stretched over the time axis so as to stand out more in this dimension (which is known to be short as the consecutive set of values span a length of time less than a predetermined minimum length of time).

In some embodiments, the method may further comprise augmenting the graphical representation of the consecutive set of values to be user interactable, such that information describing the consecutive set of values and/or the modification of the graphical representation is output responsive to the user providing an interaction action.

Accordingly, information relevant to the consecutive set of values may be provided. If the graphical representation has been modified, this may be beneficial to also indicate the actual values of the consecutive set of values so that an observer is not misled.

In exemplary embodiments, the graphical representation of the values of the physiological parameter of the subject over time may comprise a graph depicting each of the values in one of a set of value ranges arranged in a lateral direction according to a time corresponding to the value. Each value range may describe a discrete range of values of the physiological parameter, and each value range may be associated with an elevation on the display and a color corresponding to the elevation.

Thus, the invention may be applied to a collapsed view of the physiological parameter.

The physiological parameter may be at least one of a vital sign, an advanced hemodynamic parameter, a haematological parameter, and an analyte concentration. Specifically, the physiological parameter may be at least one of a heart rate, a respiration rate, a blood pressure, a cardiac output, a systemic vascular resistance, a red blood cell count, a white blood cell count, a haemoglobin concentration, a glucose concentration in whole blood, plasma or serum, and a lactate concentration in whole blood, plasma or serum.

Nevertheless, the physiological parameter may be any value that varies in time and that relates to the condition and/or health of the subject. This includes measured physiological values, as well as values generated by an algorithm and/or model based on measured physiological values, subject characteristics, etc.

According to examples in accordance with another aspect of the invention, there is provided a computer program comprising computer program code means adapted, when said computer program is run on a computer, to implement a disclosed method for displaying a graphical representation of values of a physiological parameter of a subject as a function of time.

According to further examples in accordance with an aspect of the invention, there is provided a system for displaying a graphical representation of values of a physiological parameter of a subject as a function of time, comprising:
an interface configured to receive a data series of values of the physiological parameter;
a processor configured to:
   identify a consecutive set of the values of the physiological parameter in a value range differing from a value range of the values of the physiological parameter immediately preceding and following the consecutive set of values, wherein the identified consecutive set of values span a length of time below a predetermined minimum length of time;
   analyze the consecutive set of values based on a ruleset to determine a clinical relevance of the consecutive set of values; and
   a display configured to modify the graphical representation of the consecutive set of values of the physiological parameter based on the determined clinical relevance.

These and other aspects of the invention will be apparent from and elucidated with reference to the embodiments described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 presents a typical visual interface including information of change of a plurality of physiological parameters over an 8 hour period;
Fig. 2 presents two simplified visualizations of a change of a physiological parameter over time;
Figs 3-8 provide examples of the results of modifying a graphical representation of a consecutive set of values according to examples in accordance with an aspect of the invention;
Fig. 9 presents a further augmentation of the visualization of the consecutive set of values according to another aspect of the invention;
Fig. 10 is a flow diagram of a method for displaying a graphical representation of values of a physiological parameter of a subject as a function of time according to an exemplary embodiment of the invention;
Fig. 11 is a simplified block diagram of a system for displaying a graphical representation of values of a physiological parameter of a subject as a function of time according to another embodiment of the invention; and
Fig. 12 is a simplified block diagram of a computer within which one or more parts of an embodiment may be employed.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. The mere fact that certain measures are recited in mutually different dependent claims does not indicate that a combination of these measures cannot be used to advantage.

It should be understood that the Figures are merely schematic and are not drawn to scale. It should also be understood that the same reference numerals are used throughout the Figures to indicate the same or similar parts.

Proposed concepts aim to provide schemes, solutions, concepts, designs, methods, and systems pertaining to displaying a graphical representation of values of a physiological parameter. Specifically, a consecutive set of values are identified, having values that differ/deviate from values immediately preceding and/or following the consecutive set. In other words, short term deviations of the value of the physiological parameter are identified, which may contribute to a cluttered and confusing display of the physiological parameter. Therefore, the identified consecutive set of values are analyzed with respect to a ruleset to determine a clinical relevance of the values, and the graphical representation is modified accordingly. For example, if the consecutive set of values are of low clinical relevance, then graphical parameters of the representation of the set of values may be modified to make the representation less visible to a viewer.

Examples and implementations in accordance with the invention overcome problems associated with outputting a series of measured or generated values of a physiological parameter to a screen. When the data series of values of a physiological parameter is presented in this way, short-term changes in the physiological parameter may either be unduly distracting (if of low clinical relevance) or may have a chance of being ignored/missed in error (if of high clinical relevance). In any case, high levels of cognitive effort need to be expended by the observer to properly interpret the presented data series. This problem is compounded when many different physiological parameters are provided on one display.

Accordingly, it is proposed to identify short-term changes corresponding to a series of consecutive values of the physiological parameter. These values are then analyzed in comparison to a ruleset to determine a (level of) clinical relevance of the consecutive set of values. Based on the clinical relevance, a graphical representation of the consecutive set of values is modified, thus hiding/obscuring the graphical representation or making the graphical representation stand-out, as appropriate.

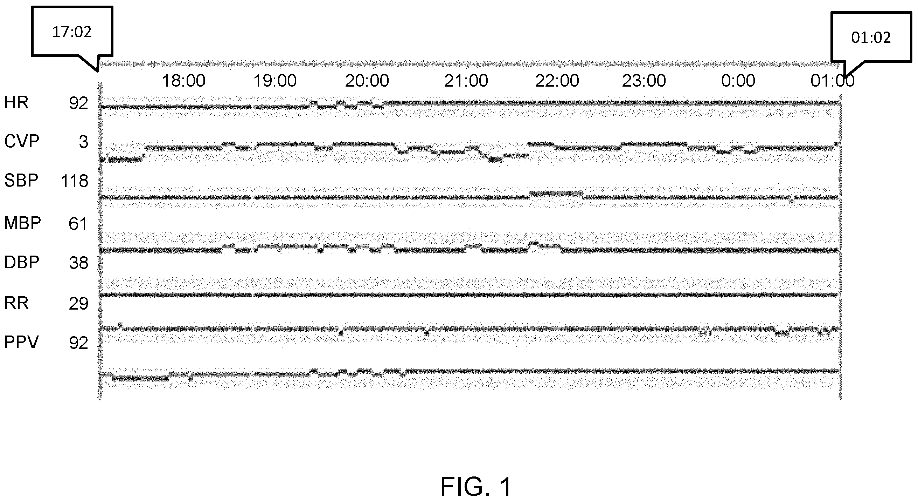

Typically, monitors/displays are utilized to present multiple physiological parameters of a subject as a function of time. Such displays form part of a typical workflow of a healthcare professional to quickly ascertain the condition of the subject, as well as their previous condition. As example of what may be displayed is shown in Fig. 1, depicting the change of multiple physiological parameters over an 8 hour period.

Depicted is a collapsed view, where the values of the physiological parameter have been classified into discrete levels, thus making it easier for a user to quickly interpret and identify periods of time of interest where the value of the physiological parameter deviates from a normal/typical value. In this collapsed view, information about exact historical values of the physiological parameter is removed, while keeping information of the parameter value's range. The range is indicated by an elevation on the screen and/or a color. For example, a middle line may be used when a parameter is in its normal range, depicted in grey, a line above and/or below that is used when the parameter is in a yellow alarm range, and a line above/below that is used when the parameter is in a red alarm range.

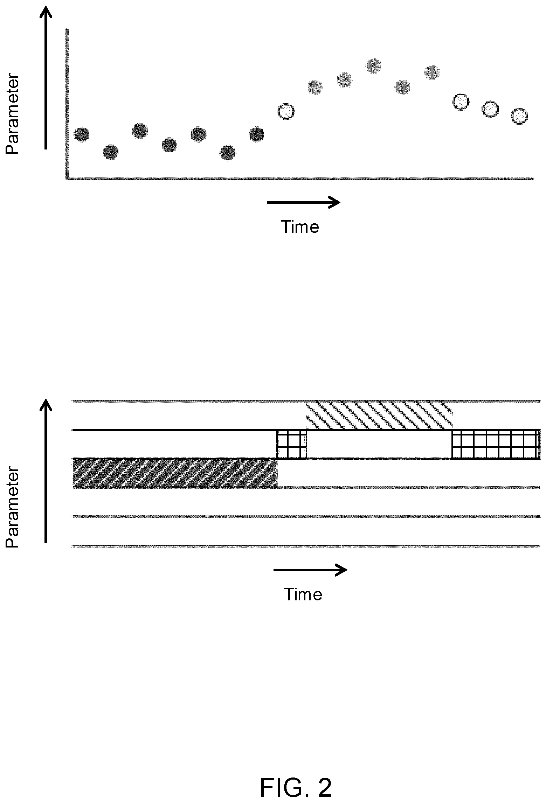

Further examples of simplified views are shown in Fig. 2. Specifically, an abstracted version of the graph for presenting values of a single physiological parameter is shown in the top graph. An equivalent dataset presented with the individual datapoints visualized is shown in the bottom graph. In particular, the bottom graph provides different shades depending on an associated data range, corresponding to an elevation on the graph.

While these methods of visualization provide a useful abstraction for the simplification of the view, this can still appear cluttered. This is particularly the case, for example, when the value of the physiological parameter rises and/or falls for short (relative to the overall length of the time displayed) length of time. Indeed, this problem applies to any graph showing progress of a physiological parameter with time - with short, sharp, deviations from the long term trend of the value of the physiological parameter leading to a cluttered display that may be difficult to interpret at a glance.

Therefore, to lessen the cognitive load on the viewer of a display of a physiological parameter, it is proposed to modify the graphical representation of sets of values of the physiological parameter that deviate briefly from recent past or future values, and that are of low clinical relevance. That is, a graphical representation of a short-term consecutive set of values may be modified based on the consecutive set's clinical relevance.

For example, if the consecutive set of values is of low clinical significance/relevance, then the graphical representation of the consecutive set of values may be modified to be less visible (e.g., by making such a visual representation similar to the visual representation of the neighboring set of values). Put another way, the view of the physiological parameter may be smoothened if short-term changes are deemed to be of low/no clinical relevance.

In contrast, if it is found that a short term deviation of the value of the physiological value is of great/high clinical relevance/significance, the representation of such values may be modified to be made more visible. For example, such a representation of the significant values may be made larger, or otherwise highlighted so that a user can quickly identify the change. As a result, short-term clinically relevant deviations may be identified in a straightforward and fast manner by a user.

Therefore, embodiments of the invention declutter the graphical representation of values of physiological parameter(s) of a subject by reducing the visibility of all short-term changes of values that are of little clinical relevance or are clinically irrelevant. In this way, such short-term changes will not distract a viewer. Alternatively or additional embodiments, causes the visual representation of short term highly clinically relevant changes to be more prominent, so that the attention of a viewer may be drawn.

An implementation of this disclosure may provide:
(i) A display for presenting a visual representation of values of one or more physiological parameters of a subject as a function of time. For example, a view as in Fig. 1 may be provided;
(ii) A processor for:
   (a) splitting a data series of values of the physiological parameter into data segments, with each data segment containing all consecutive data belonging to the same range. The data segment may end when the following value in the data series is part of another range;
   (b) determining whether a data segment has values spanning less than a threshold duration;
   (c) upon identifying a data segment spanning a shorter time period than the threshold duration, determining whether the values of the physiological parameter in the data segment are clinically relevant; and
   (d) modifying the visual representation of values of the physiological data in the data segment so that they stand out when the values are highly clinically relevant, and stand out less when the values are less (or not) clinically relevant.

Generally, making the visual representation of the values stand out may be achieved by widening its visualization (i.e. making it start from an earlier time than when it originally started and/or end at a later time than when it originally ended). Alternatively or additionally, the visual representation may be made a different color, pattern, or brightness to the visual representation of neighboring values to draw the attention of a viewer.

Making a data segment stand out less may be achieved by matching the color, pattern, brightness, and/or elevation of the visual representation of the values within the data segment with said visualization parameters of the values immediately preceding and/or following the data segment.

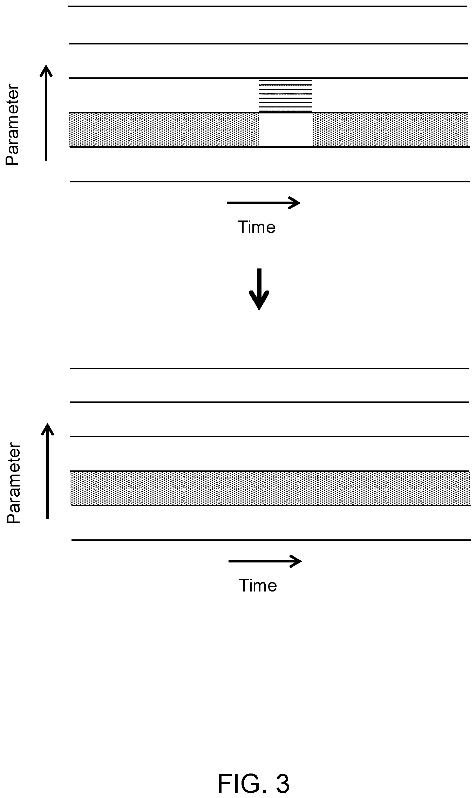

Methods for making the visual representation of the values will be elucidated in greater detail in reference to Figs. 3-8.

To determine whether a set of values of a physiological parameter is of clinical relevance, significance and/or importance, a ruleset may be provided. The ruleset may include the following categories of rules (or combinations thereof):
(i) Rules that relate to alarm settings or goal settings;
(ii) Rules that relate to the surrounding environment/context of the subject (e.g. time of the day, location);
(iii) Rules that relate to interventions that have been performed on the subject;
(iv) Rules that relate to actions of that have been performed by the subject; and
(v) Rules that relate to the timescale of the physiological parameter being displayed, for example as set by a clinician.

Of course, the ruleset may be decided by individual healthcare providers, caregivers, or other users to suit their preference and requirements.

For example, the ruleset could include the following rules:
(a) For heart rate, a change to a higher range of values and shortly after that back to the lower range of values is of high clinical relevance when it is during the night, but of low clinical relevance when it is during daytime;
(b) A change of a physiological parameter into an alarm range and shortly after that back to normal range for the physiological parameter is of high clinical relevance when treatment was required to return the value of the physiological parameter back into the normal range, but is not of importance if the physiological parameter spontaneously returns back to the normal range;
(c) When the subject is moving (e.g. detected by an accelerometer on the subject, from a signal to noise ratio of the sensor of the physiological parameter, or from changes in the physiological parameter that are too sudden to be physiologically true for a subject at rest), short term changes in physiological parameters (e.g., heart rate) are of low clinical relevance;
(d) When cardiac output shows an increase with a maximum value of 0.3 l/min after ~2 min and a decrease back to the original value after 10 min then this is classified as a fluid challenge (alternatively, an electronic medical record could be analyzed to detect whether there was a fluid challenge). This is of high clinical relevance when the viewer is observing a small timescale of the physiological parameter on the display (e.g. when they have set the time axis of the display of the physiological parameter to cover up to 30 minutes), because then the viewer is likely to be interested in determining whether the subject is a so-called "responder" or "non-responder". Conversely, this deviation may be of low clinical relevance when the viewer is observing a larger timescale of the physiological parameter on the display (e.g. when they have set the times axis of the display of the physiological parameter to cover more than 4 hours), because then the viewer is likely interested in observing the overall history/trend of the subject, and disturbances should be removed.
(e) A short-term change in a value of a physiological parameter into an alarm range may be of low clinical relevance when the corresponding alarm was silenced.

It is worth noting that the identification of the consecutive set of values spanning a length of time below a threshold length of time may be rule-specific. That is, the identification of a short-term deviation of a value of a physiological parameter may be dependent on the rule.

In addition to a rule set, a user may provide a manual input regarding the relevance of a consecutive set of values of the physiological parameter. For example, the clinician may provide a single click, or short tap on a visual representation of the data segment on a display using a mouse to indicate that the data segment is of low clinical relevance, and a double tap on the visual representation of the data segment using the mouse to indicate that the data segment is of high clinical relevance.

Ways in which the visual representation of the consecutive set of values of the physiological parameter may be modified will now be described in reference to Figs. 3-8. It is worth noting that the modifications are all to visual representation of the set of values having been discretized into value ranges. However, this is for the sake of simplicity and brevity. The same principles may be applied to a continuous representation of the set of values.

Fig. 3 presents a short-term consecutive set of values 10 that are determined to be of low clinical relevance. Accordingly, graphical parameters of the visual representation of those values are modified to be the same as the values immediately preceding and following the consecutive set of values. In this case, an elevation parameter, color and/or pattern parameter are changed. In other words, the short-term set of values of low clinical relevance are effectively omitted from the display of the data series.

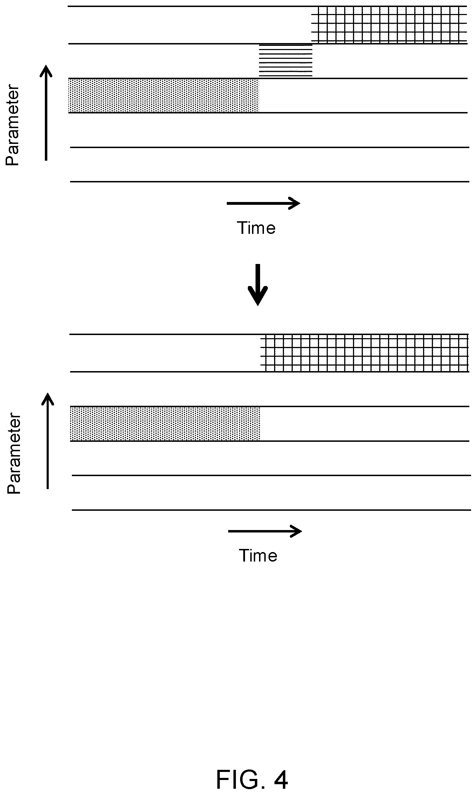

Fig. 4 presents a situation in which the value of the physiological parameter has increased by a significant amount. In changing, there was a short amount of transition time between the lower range of values and an upper range of values. In this case, the short-term consecutive set of values 10 during transition are determined to be of low clinical relevance, and therefore are omitted from view similarly to Fig. 3 to make the display less cluttered. Accordingly, graphical parameters of the visual representation of those values are modified to be the same as the values either immediately preceding or immediately following the consecutive set of values (or could be a mix of both). In this case, an elevation parameter, color and/or pattern parameter are changed. In other words, the short-term set of values of low clinical relevance are effectively omitted from the display of the data series.

Alternatively, it may be determined that although the consecutive set of values 10 are of low clinical relevance, no changes may be made as the values were simply in transition. This may depend on the ruleset and/or preferences of a user/viewer.

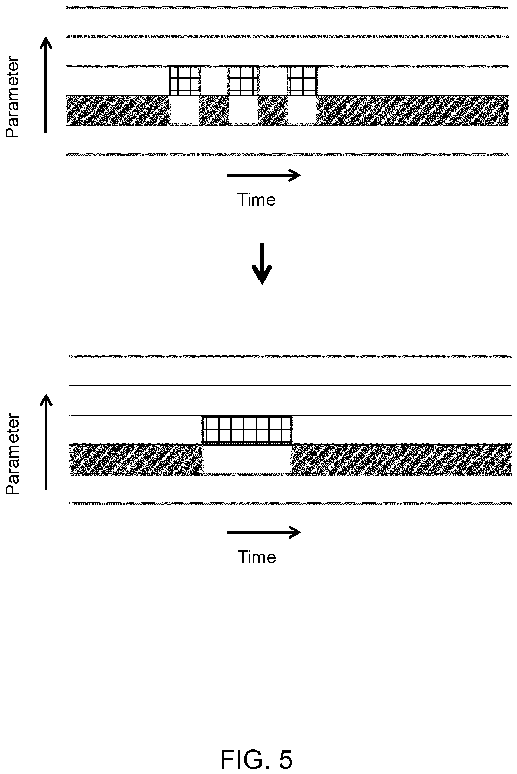

Fig. 5 presents a situation in which the value of the physiological parameter oscillates between two values repeatedly at a relatively high frequency compared to the timescale displayed. Accordingly, graphical parameters of the visual representation of various sets of consecutive values are modified to be the same as the values immediately preceding and following the values, therefore removing the oscillation that is of low clinical relevance.

Furthermore, the duration of the modified block in the upper range in Fig. 5 was made to match the cumulative duration of the segments in the upper range before modification. In other words, the amount of time the physiological parameter belongs to the upper range in the original visual representation matches the amount of time the physiological parameter is represented as belonging to the upper range in the modified visual representation.

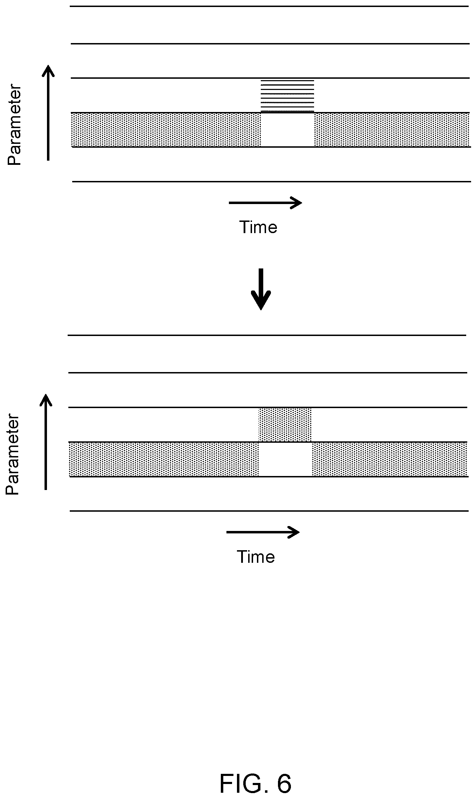

Fig. 6 presents the situation of Fig. 3. However, in this case not all of the graphical parameters are modified to be the same as the graphical parameters of the visual representation neighboring values. Indeed, in this case only the color/pattern has been modified, resulting in a less cluttered view (but still displaying the change if the viewer looks closely.

In other words, if the consecutive set of values is of low clinical relevance, the graphical parameter of elevation may remain the same, but with the color/pattern of the values immediately preceding and/or following. This graphical parameter(s) that may be matched or stay the same responsive to the determination of low clinical relevance may be set by the viewer/user. Alternatively, the timescale of the time series of data of the physiological parameter being displayed may dictate the number/which of the graphical parameters to alter. For example, when the timescale is short, there may be no matching, whereas when the timescale is long, all graphical parameters may be matched (because the viewer may be more likely to be interested in the long term trend when choosing a larger timescale).

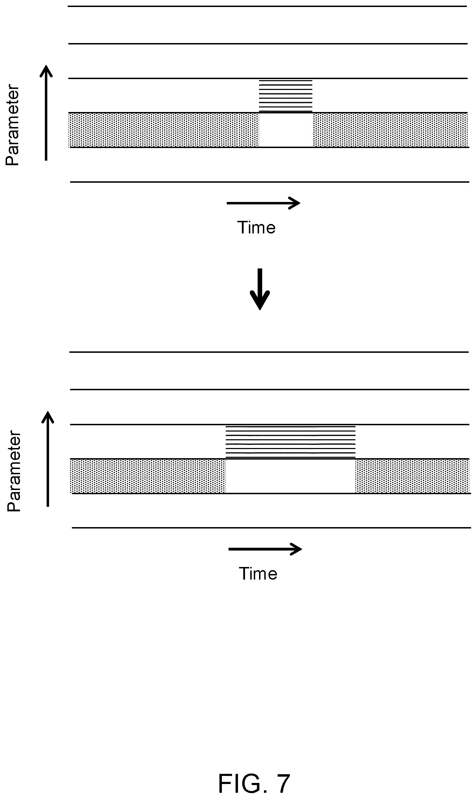

Fig. 7 presents a short-term consecutive set of values 10 that are determined to be of high clinical relevance. Accordingly, graphical parameters of the visual representation of those values are modified so that the set of values appears larger on the display. That is, the set of values are displayed over a longer period of time so that they are easier to be seen. Alternatively, the graphical parameter of those values may be modified to stand out in other ways, such as a vibrant color or increased brightness.

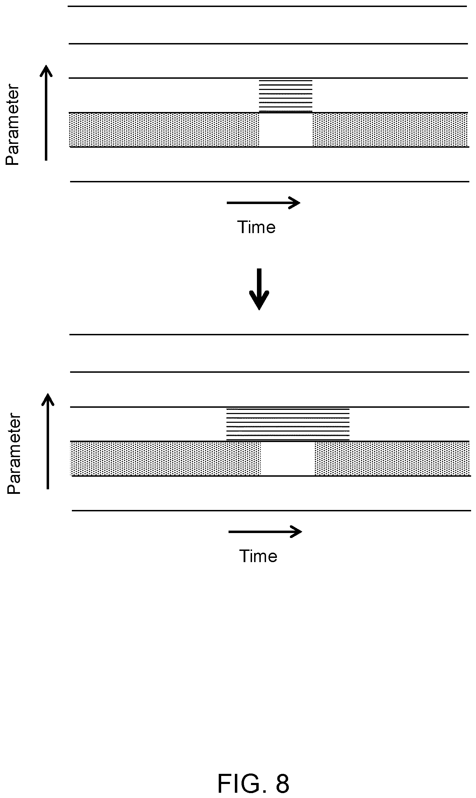

Fig. 8 also presents a short-term consecutive set of values 10 that are determined to be of high clinical relevance. However, instead of reducing the size of the graphical representation of values immediately preceding and/or following the consecutive set of values 10, only the representation of the consecutive set of values is increased.

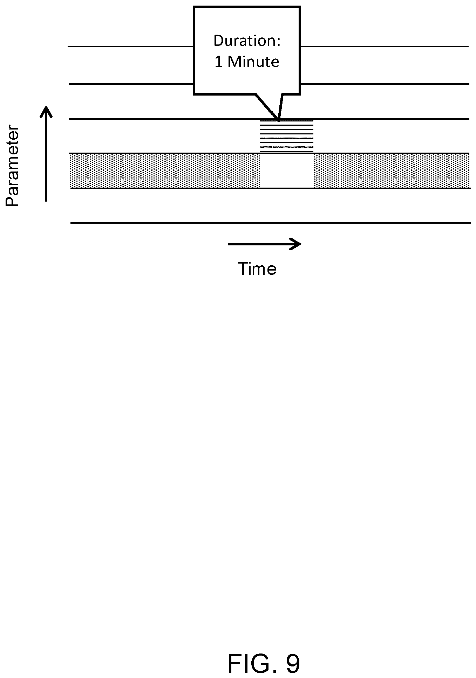

Fig. 9 presents a further feature that may be implemented in some examples of the disclosure. In this case, the visual representation of the short-term consecutive set of values is augmented by adding more details about the set. The viewer may receive this information by providing an input, such as clicking on, tapping or hovering over the graphical representation.

In this case, even if the size of the consecutive set of values was increased (such as in the situation described in reference to Fig. 7 and Fig. 8) the viewer can still get the information representative of the original data.

Finally, the viewer may also be provided with an option to toggle on/off the changes to the representation of the values. This may enable a viewer to appreciate that they are not necessarily viewing the complete dataset, and use their own judgement if required. Additionally or alternatively, a part of the display may indicate time periods corresponding to the consecutive set of values that have been modified.

To reiterate, while examples provided are related to the collapsed view of the data series of values of the physiological parameter, the disclosed principles can be applied to other visualizations of the data series. For example, the data series may be represented as a graph with each value being plotted as a datapoint on a vertical scale, without mapping it first to a range.

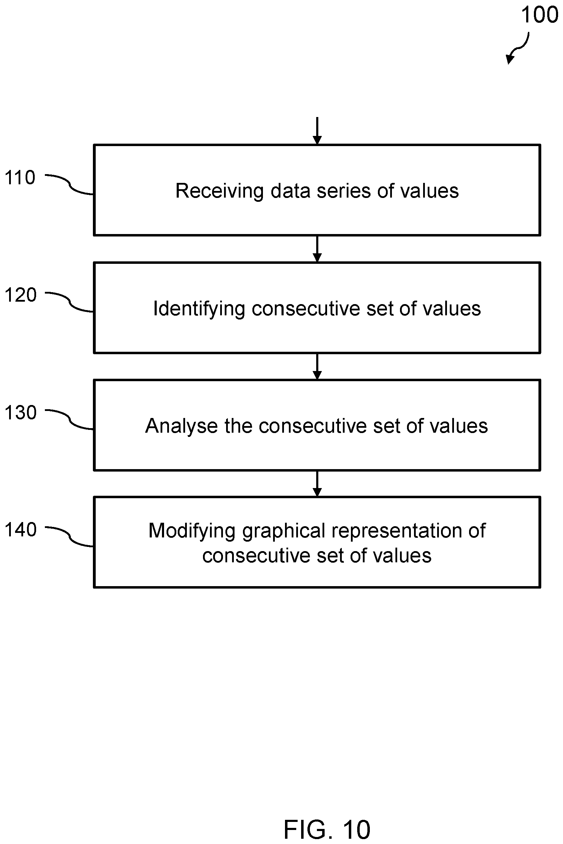

Fig. 10 is a flow diagram of a method for displaying a graphical representation of values of a physiological parameter of a subject as a function of time according to an exemplary embodiment of the invention.

In step 110, a data series of values of the physiological parameter is received/obtained.

The data series of values of the physiological parameter may be any sequence of values of the physiological parameter in time. The values may be a continuous, real time, stream of values. Alternatively, the values may be a series of historical values generated previously.

The values may be measured values (i.e. taken directly from sensors), or may be generated/calculated values (i.e. obtained from an algorithm/model). The values may be taken directly from sensors/data generators, or may be obtained from a database of values (e.g., electronic medical records of the subject).

The physiological parameter may be at least one of a vital sign, an advanced hemodynamic parameter, a haematological parameter, and an analyte concentration. For example, the physiological parameter may be at least one of a heart rate, a respiration rate, a blood pressure, a cardiac output, a systemic vascular resistance, a red blood cell count, a white blood cell count, a haemoglobin concentration, a glucose concentration in whole blood, plasma or serum, and a lactate concentration in whole blood, plasma or serum. It is worth noting that this is not considered limiting, and any parameter relate to the health and/or condition of the subject may be considered a physiological parameter.

To reiterate, as well as measured values, the physiological parameter may be a value generated using an algorithm and/or model. The data series of values of the physiological parameter may be based on values of (one or more) measured physiological parameters, and optionally other relevant parameters (e.g., demographics, device settings, etc.). Any time-varying parameter related to the condition and/or health of the subject may be presented graphically as a function of time, and have a related clinical relevance, and therefore may be a physiological parameter within the definition of the invention.

For example, the physiological parameter may be a hemodynamic stability index (HSI) indicative of the likelihood of a subject becoming hemodynamically stable. The HSI may not be measured directly, but may be based on measured physiological parameter values, patient characteristics (e.g., subject age) and ventilator settings. Of course, the hemodynamic stability index is merely one example of a physiological parameter having values generated using an algorithm/model. Other predictive values may also be plotted over time and therefore provide the data series of values.

In step 120, a consecutive set of the values of the physiological parameter in a value range differing from a value range of the values of the physiological parameter immediately preceding and following the consecutive set of values are identified. The identified consecutive set of values span a length of time below a predetermined minimum length of time.

That is, a set of values in the data series of values are identified that fulfil two criteria. Firstly, the consecutive set of values must differ from the values of the parameter directly preceding and following the set, in that they belong to a different value range. For example, the consecutive series of values may be a spike/trough in the data series. It is worth noting that the consecutive set of values do not need to be the same value, but they must belong within the same/similar range of values.

Secondly, the consecutive set of values must only span a length of time up to a predetermined minimum length of time. In other words, the consecutive set of values must correspond to the physiological parameter of the subject for up to a given period of time. The predetermined minimum length of time may be based on protocols, literature, user preferences, and default values.

In specific embodiments, the predetermined minimum length of time may be based on the length of time spanned by the displayed values of the physiological parameter. For example, the predetermined minimum length of time may be relative to the time span (i.e. time resolution) of the displayed values of the physiological parameter.

Moreover, the predetermined minimum length of time may also be dependent on the physiological parameter being displayed. Thus, the predetermined minimum length of time may be different for different physiological parameters being displayed. For example, the predetermined minimum length of time associated with a heart rate of the subject may be longer/shorter than the predetermined minimum length of time associated with a glucose level of the subject.

In some cases, the consecutive set of values may include only one value/data point. This may occur when the physiological parameter instantaneously deviates from neighboring values such that a value is the only value belonging to the value range. Of course, it may be beneficial to analyze the clinical relevance of such an isolated value, and adapt the graphical representation of this value accordingly.

In brief, the consecutive set of values correspond to a short-term deviation in the value of the physiological parameter.

In particular, identifying the consecutive set of values may comprise obtaining a set of value ranges, each describing a discrete numerical range of values for the physiological parameter. Then, the values of the physiological parameter may be divided/segmented into a plurality of data segments, each data segment comprising a consecutive set of values falling within one of the sets of value ranges. Accordingly, at this stage there is provided a series of data segments consecutive in time, each neighboring data segment correspond to different data ranges.

Specifically, each of the set of value ranges may correspond to a different alarm setting related to the physiological parameter. For example, the set of the value ranges comprises at least one value range corresponding to each of a normal range of the physiological parameter, an elevated range of the physiological parameter, a depressed range of the physiological parameter, a dangerously high range of the physiological parameter, and a dangerously low range of the physiological parameter.

Thus, for each data segment, it is determined whether the data segment spans a length of time below the predetermined minimum length of time. Data segments spanning a length of time below the predetermined minimum length of time are then identified as a consecutive set of values for further processing.

In step 130, the consecutive set of values are analyzed based on a ruleset to determine a clinical relevance of the consecutive set of values. That is, the values are analyzed/interpreted by an appropriate algorithm/model to determine a (level of) clinical relevance.

Specifically, the analysis of the set of values may comprise comparing the consecutive set of values to an alarm condition describing values indicative of a potential health risk for the subject, optionally wherein the alarm condition is based on a condition of the subject, a situation of the subject, previous interventions performed on the subject, previous actions performed by the subject and/or a length of time spanned by the displayed values of the physiological parameter.

In other words, the analysis may be performed by simply comparing the set of values to a benchmark set of values describing a potential health risk (and therefore clinical relevance) to the subject. Such a benchmark set of values can be based on the condition of the subject (e.g., any known health issues/contributing factors), the situation of the subject (e.g., progress/deterioration in the condition of the subject), intervention performed on the subject (e.g., medication provided that may induce a change in expected ranges of a value of the physiological parameter), actions performed by the subject (e.g., movements of the subject), and the length of time spanned by the displayed values.

Further/alternative considerations include:
(i) comparing the consecutive set of values to an alarm condition describing values indicative of a potential health risk for the subject. For example, if the consecutive set of values correspond to a relevant alarm or target value of the physiological parameter, then they may be considered of clinical relevance/significance;
(ii) analyzing a surrounding environment of the subject corresponding to the acquisition of the consecutive set of values. Indeed, conditions in the surrounding environment describing a context of the subject may be analyzed. For example, if the surrounding environment is briefly noisy, then this may explain changes in physiological parameters associated with stress, and therefore brief deviations in these physiological parameters may be considered of low clinical relevance;
(iii) analyzing a user action responsive to an alarm associated with the consecutive set of values. For example, if a user silenced an alert indicative of a sudden change in the physiological parameter, then this may be indicative of the deviation being of low clinical relevance/interest;
(iv) analyzing a subject action performed by the subject during acquisition of the consecutive set of values. If the subject was performing a certain action (e.g., standing up), then this may be associated with a change in a physiological parameter (e.g., a brief increase in heart rate), which may then be considered of low clinical relevance. In contrast, if the subject did not perform any actions (e.g., was sleeping) then brief changes in physiological parameter may be of high clinical relevance/interest;
(v) analyzing a length of time spanned by the displayed values of the physiological parameter. For example, a relevance of a short-term change of the physiological parameter may be dependent on the timescale being observed by a user (i.e., zoom settings selected by the user). In other words, if the user has chosen to observe a short time scale, they may be looking for short-term changes. Instead, if the user has chosen to observe a large time scale, they may not be concerned with any short-term changes.

Of course, other factors may be taken into consideration when assessing the clinical relevance of the consecutive set of values. The clinical relevance may be dictated by the requirements of specific users, for example.

In step 140, the graphical representation of the consecutive set of values of the physiological parameter is modified based on the determined clinical relevance.

The graphical representation may be modified to make the values of the physiological parameter more prominent responsive to determining that the values are of high clinical relevance, or to obscure/hide the values of the physiological parameter responsive to determining that the values are of low clinical relevance. Essentially, the modification is performed to generate a graphical representation that is more appropriate for quick and simple observation of the physiological parameter..

Specifically, the graphical representation of each of the values may be defined by a configurable set of graphical parameters. The configurable set of graphical parameters may comprise at least one of a color parameter, pattern parameter, elevation parameter, brightness parameter, and/or size parameter. Each of these parameters determine how the graphical/visual representation of the values of the physiological parameter appear on the screen.

Responsive to determining that the consecutive set of values are of low clinical relevance, step 140 may comprise modifying at least one of the graphical parameters of the consecutive set of values to be substantially equal to the graphical parameters of at least one of the values immediately preceding or following the consecutive set of values.

Accordingly, the graphical representation of the consecutive set of values may appear to be more similar to the graphical representation of the neighboring values - meaning that it is less likely to distract/draw the attention of an observer.

When the value preceding and the value following the consecutive set are substantially the same, the graphical parameters of the consecutive set of values may be modified to be the same as (or an average of) both of the graphical parameters of the values immediately preceding and following the consecutive set of values.

Responsive to determining that the consecutive set of values of the physiological parameter of high clinical relevance, step 140 may comprise increasing a size parameter defining a size of the graphical representation of the consecutive set of values. Increasing the size parameter of the consecutive set of values may comprise enlarging a length of the time axis of the graphical representation corresponding to the consecutive set of values.

In any case, when the consecutive set of values are considered of high clinical relevance, then the graphical representation may be modified to make the values stand out.

Finally, the method 100 may further comprise an additional, optional, step of augmenting/modifying the graphical representation of the consecutive set of values to be user interactable. Thus, responsive to the user providing an interaction action, information describing the consecutive set of values and/or the modification of the graphical representation is output. This may be in the form of visual or audio output.

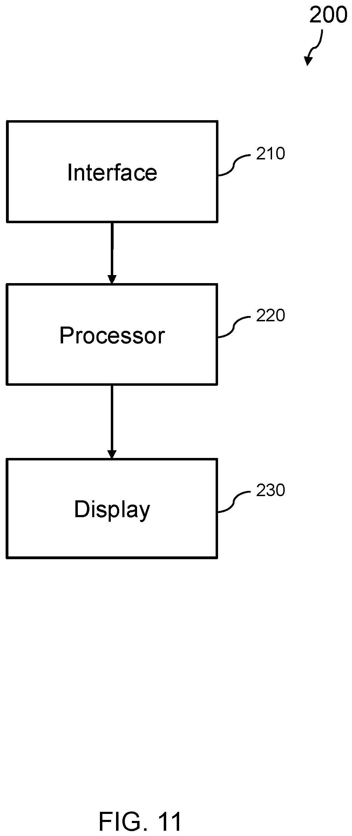

Fig. 11 is a simplified block diagram of a system for displaying a graphical representation of values of a physiological parameter of a subject as a function of time according to another embodiment of the invention. The system comprises an interface 210, a processor 220, and a display 230.

The interface 210 is configured to receive a data series of values of the physiological parameter, as described above in reference to step 110 of method 100.

The processor 220 is configured to process and analyze the data series of values of the physiological parameter. Specifically, the processor is at least configured to identify a consecutive set of the values of the physiological parameter in a value range differing from a value range of the values of the physiological parameter immediately preceding and following the consecutive set of values. The identified consecutive set of values span a length of time below a predetermined minimum length of time.

Further, the processor 220 is configured analyze the consecutive set of values based on a ruleset to determine a clinical relevance of the consecutive set of values.

Of course, the processor 220 may also be configured to process and analyze the data series to identify consecutive sets of values and assess their relevance according to the methods disclosed in reference to steps 120 and 130 of method 100.

Finally, the display 230 is configured to modify the graphical representation of the consecutive set of values of the physiological parameter based on the determined clinical relevance.

Accordingly, the above described system may be used to improve means for presenting a visual representation of a physiological parameter as a function of time by taking into account the clinical relevance of short-term deviations of the physiological parameter when producing and/or modifying the visual representation. Such a system may be integrated with existing visualization systems, or be provided as a part of new visualization systems.

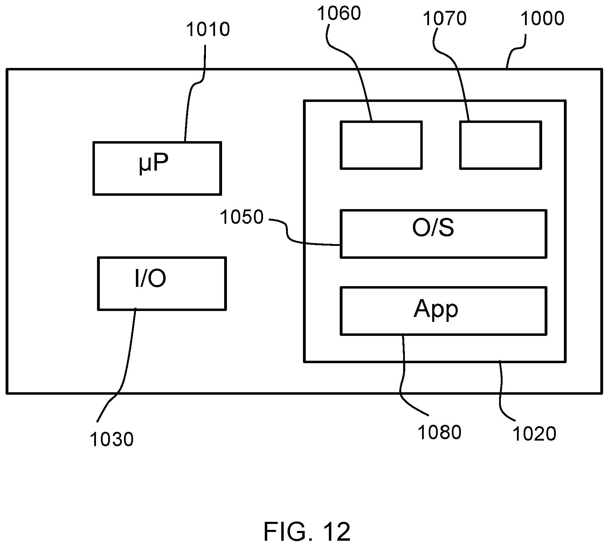

Fig. 12 illustrates an example of a computer 1000 within which one or more parts of an embodiment may be employed. Various operations discussed above may utilize the capabilities of the computer 1000. For example, one or more parts of a system for acquiring an input from a user to control an interface may be incorporated in any element, module, application, and/or component discussed herein. In this regard, it is to be understood that system functional blocks can run on a single computer or may be distributed over several computers and locations (e.g. connected via internet).

The computer 1000 includes, but is not limited to, smart phones, PCs, workstations, laptops, PDAs, palm devices, servers, storages, and the like. Generally, in terms of hardware architecture, the computer 1000 may include one or more processors 1010, memory 1020 and one or more I/O devices 1030 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 1010 is a hardware device for executing software that can be stored in the memory 1020. The processor 1010 can be virtually any custom made or commercially available processor, a central processing unit (CPU), a digital signal processor (DSP), or an auxiliary processor among several processors associated with the computer 1000, and the processor 1010 may be a semiconductor-based microprocessor (in the form of a microchip) or a microprocessor.

The memory 1020 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 1020 may incorporate electronic, magnetic, optical, and/or other types of storage media. Note that the memory 1020 can have a distributed architecture, where various components are situated remote from one another, but can be accessed by the processor 1010.

The software in the memory 1020 may include one or more separate programs, each of which comprises an ordered listing of executable instructions for implementing logical functions. The software in the memory 1020 includes a suitable operating system (O/S) 1050, compiler 1060, source code 1070, and one or more applications 1080 in accordance with exemplary embodiments. As illustrated, the application 1080 comprises numerous functional components for implementing the features and operations of the exemplary embodiments. The application 1080 of the computer 1000 may represent various applications, computational units, logic, functional units, processes, operations, virtual entities, and/or modules in accordance with exemplary embodiments, but the application 1080 is not meant to be a limitation.

The operating system 1050 controls the execution of other computer programs, and provides scheduling, input-output control, file and data management, memory management, and communication control and related services. It is contemplated by the inventors that the application 1080 for implementing exemplary embodiments may be applicable on all commercially available operating systems.

Application 1080 may be a source program, executable program (object code), script, or any other entity comprising a set of instructions to be performed. When a source program, then the program is usually translated via a compiler (such as the compiler 1060), assembler, interpreter, or the like, which may or may not be included within the memory 1020, so as to operate properly in connection with the O/S 1050. Furthermore, the application 1080 can be written as an object oriented programming language, which has classes of data and methods, or a procedure programming language, which has routines, subroutines, and/or functions, for example but not limited to, C, C++, C#, Pascal, Python, BASIC, API calls, HTML, XHTML, XML, ASP scripts, JavaScript, FORTRAN, COBOL, Perl, Java, ADA, .NET, functional programming and the like.

The I/O devices 1030 may include input devices such as, for example but not limited to, a mouse, keyboard, scanner, microphone, camera, etc. Furthermore, the I/O devices 1030 may also include output devices, for example but not limited to a printer, display, etc. Finally, the I/O devices 1030 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 1030 also include components for communicating over various networks, such as the Internet or intranet.

If the computer 1000 is a PC, workstation, intelligent device or the like, the software in the memory 1020 may further include a basic input output system (BIOS) (omitted for simplicity). The BIOS is a set of essential software routines that initialize and test hardware at start-up, start the O/S 1050, and support the transfer of data among the hardware devices. The BIOS is stored in some type of read-only-memory, such as ROM, PROM, EPROM, EEPROM or the like, so that the BIOS can be executed when the computer 800 is activated.

When the computer 1000 is in operation, the processor 1010 is configured to execute software stored within the memory 1020, to communicate data to and from the memory 1020, and to generally control operations of the computer 1000 pursuant to the software. The application 1080 and the O/S 1050 are read, in whole or in part, by the processor 1010, perhaps buffered within the processor 1010, and then executed.

When the application 1080 is implemented in software it should be noted that the application 1080 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 1080 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The methods described in relation to Fig. 10, and the systems described in relation to Fig. 11, may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagram of Fig. 11 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

## Claims

1. A method (100) for displaying a graphical representation of values of a physiological parameter of a subject as a function of time, comprising:
receiving (110) a data series of values of the physiological parameter;
identifying (120) a consecutive set of the values of the physiological parameter in a value range differing from a value range of the values of the physiological parameter immediately preceding and following the consecutive set of values, wherein the identified consecutive set of values span a length of time below a predetermined minimum length of time;
analyzing (130) the consecutive set of values based on a ruleset to determine a clinical relevance of the consecutive set of values; and
modifying (140) the graphical representation of the consecutive set of values of the physiological parameter based on the determined clinical relevance.

2. The method of claim 1, wherein identifying (120) the consecutive set of values of the physiological parameter comprises:
obtaining a set of value ranges, each describing a discrete numerical range of values for the physiological parameter;
dividing the values of the physiological parameter into a plurality of data segments, each data segment comprising a consecutive set of values falling within one of the sets of value ranges; and
for each data segment, determining whether the data segment spans a length of time below the predetermined minimum length of time.

3. The method of claim 2, wherein each of the set of value ranges corresponds to a different alarm setting related to the physiological parameter, and optionally wherein the set of the value ranges comprises at least one value range corresponding to each of a normal range of the physiological parameter, an elevated range of the physiological parameter, a depressed range of the physiological parameter, a dangerously high range of the physiological parameter, and a dangerously low range of the physiological parameter.

4. The method of any of claims 1-3, wherein analyzing (130) the consecutive set of values to determine the clinical relevance comprises at least one of
comparing the consecutive set of values to an alarm condition describing values indicative of a potential health risk for the subject;
comparing the consecutive set of values to a goal condition describing target values of the physiological parameter of the subject;
analyzing a surrounding environment of the subject corresponding to the acquisition of the consecutive set of values;
analyzing a user action responsive to an alarm associated with the consecutive set of values;
analyzing a subject action during acquisition of the consecutive set of values; and
analyzing a length of time spanned by the displayed values of the physiological parameter.

5. The method of any of claims 1-4, wherein the graphical representation of each of the values is defined by a configurable set of graphical parameters, and wherein responsive to determining that the consecutive set of values are of low clinical relevance, modifying (140) the graphical representation of the consecutive set of values comprises modifying at least one of the graphical parameters of the consecutive set of values to be substantially equal to the graphical parameters of at least one of the values immediately preceding or following the consecutive set of values.

6. The method of claim 5, further comprising:
determining whether the values immediately preceding and following the consecutive set of values differ by less than a threshold amount; and
responsive to determining that the values immediately preceding and following differ by less than the threshold amount, modifying the at least one of the graphical parameters comprises modifying at least one of the graphical parameters of the consecutive set of values to be substantially equal to the graphical parameters of both of the values immediately preceding and following the consecutive set of values.

7. The method of claims 5 or 6, wherein configurable set of graphical parameters comprises at least one of a color parameter, pattern parameter, elevation parameter, brightness parameter, and/or size parameter.

8. The method of any of claims 1-7, wherein responsive to determining that the consecutive set of values of the physiological parameter of high clinical relevance, modifying (140) the graphical representation of the consecutive set of values comprises increasing a size parameter defining a size of the graphical representation of the consecutive set of values.

9. The method of claim 8, wherein increasing the size parameter of the consecutive set of values comprises enlarging a length of the time axis of the graphical representation corresponding to the consecutive set of values.

10. The method of any of claims 1-9, further comprising augmenting the graphical representation of the consecutive set of values to be user interactable, such that information describing the consecutive set of values and/or the modification of the graphical representation is output responsive to the user providing an interaction action.

11. The method of any of claims 1-10, wherein the predetermined minimum length of time is based on the length of time spanned by the displayed values of the physiological parameter.

12. The method of any of claims 1-11, wherein the graphical representation of the values of the physiological parameter of the subject over time comprises a graph depicting each of the values in one of a set of value ranges arranged in a lateral direction according to a time corresponding to the value, each value range describing a discrete range of values of the physiological parameter, and wherein each value range is associated with an elevation on the display and a color corresponding to the elevation.

13. The method of any of claims 1-12, wherein the physiological parameter is at least one of a vital sign, an advanced hemodynamic parameter, a haematological parameter, and an analyte concentration, and optionally wherein the physiological parameter is at least one of a heart rate, a respiration rate, a blood pressure, a cardiac output, a systemic vascular resistance, a red blood cell count, a white blood cell count, a haemoglobin concentration, a glucose concentration in whole blood, plasma or serum, and a lactate concentration in whole blood, plasma or serum.

14. A computer program comprising computer program code means adapted, when said computer program is run on a computer, to implement the method of any of claims 1-13.

15. A system (200) for displaying a graphical representation of values of a physiological parameter of a subject as a function of time, comprising:
an interface (210) configured to receive a data series of values of the physiological parameter;
a processor (220) configured to:
identify a consecutive set of the values of the physiological parameter in a value range differing from a value range of the values of the physiological parameter immediately preceding and following the consecutive set of values, wherein the identified consecutive set of values span a length of time below a predetermined minimum length of time;
analyze the consecutive set of values based on a ruleset to determine a clinical relevance of the consecutive set of values; and
a display (230) configured to modify the graphical representation of the consecutive set of values of the physiological parameter based on the determined clinical relevance.
